# EUROPEAN PATENT APPLICATION

(11) **EP 2 143 432 A1**
(43) Date of publication of application: **13.01.2010**
(21) Application number: 08160239.3
(22) Date of filing: 11.07.2008
(51) Int. Cl.: A61K 31/565, A61K 31/567, A61P 35/00

(54) **9-alpha estratriene derivatives as ER-beta selective ligands for the prevention and treatment of intestinal cancer**

(71) Applicant: Bayer Schering Pharma Aktiengesellschaft, 13353 Berlin (DE)
(72) Inventor: Fritzemeier, Karl-Heinrich, 13505, Berlin (DE); Diel, Patrick, 50933, Köln (DE); Hertrampf, Torsten, 50933, Köln (DE)

(57) **Abstract**

Use of 9α-estra-1,3,5(10)-triene derivatives of formula (I) 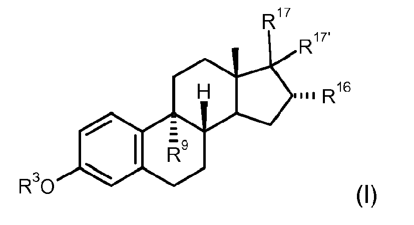 for the prevention and treatment of intestinal cancer, in particular adenoma and adenocarcinoma of the duodenum, ileum, colon, and rectum.

## Description

### Technical field

The present invention refers to the use of 9α-estra-1,3,5(10)-triene derivatives of formula (I) 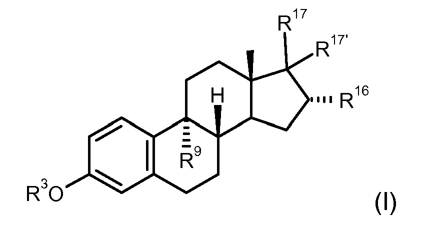 in which radicals R³, R⁹, R¹⁶, R¹⁷and R^{17'}, independently of one another, have the meaning as defined in the disclosure of the invention below, for the prevention and treatment of intestinal cancer, in particular adenoma and adenocarcinoma of the duodenum, ileum, colon, and rectum.

More in detail, the present invention refers to the use of 9α-Vinyl-estra-1,3,5(10)-triene-3,16α-diol for the prevention and treatment of intestinal cancer, in particular adenoma and adenocarcinoma of the duodenum, ileum, colon, and rectum. Further aim of the present invention is a medicament comprising a 9-alpha estratriene derivative of general formula (I), in particular 17β-fluoro 9α-vinyl- estra-1,3,5(10)-triene-3,16a-diol for the prevention or treatment of intestinal cancer, in particular adenoma or adenocarcinoma of the duodenum, ileum, colon and rectum.

### Background art

Postmenopausal hormone replacement therapy (HRT) mediates protective effects in the intestinal tract and may effectively counteract tumour development (1; 2; 3). Thus, long-term supplementation of postmenopausal women with estrogen plus progestin results in a lower incidence of colorectal cancer (CRC) (4; 5; 6). In recent years studies with estrogen receptor knockout mice (ERKOs), and those performed with ER subtype-specific agonists, have heightened interest in novel estrogen targets in the body (7), like cardiovascular system, prostate, skeletal muscle and intestinal epithelium.

Thus, unlike classical estrogen sensitive tissues like uterus and mammary gland, where ERα is mainly expressed, epithelial cells of the digestive tract express predominantly ERβ (8; 9), implicating a major importance of this ER subtype in small intestine and colon.

Interestingly, several studies have shown that in contrast to expression of ERα, ERβ expression is significantly reduced in colon tumour cells and colon cancer cell lines (9; 10; 11; 12). The potential role for ERβ-specific ligands in prevention of CRC, insight to the importance of ERβ-specific signalling for maintenance of colonic tissue homeostasis (13), and the epidemiological differences in occurrence of CRC in eastern and western populations (14) consequently led to investigations of the potential role of phytoestrogens in prevention of CRC.

Furthermore, studies performed with ERβKOs, suggest that ERβ-specific agonists and ERβ-selective phytoestrogens like genistein (GEN) and coumestrol (COU) may serve as potential regulators of intestinal tissue homeostasis (3)

Phytoestrogens, including the soy isoflavone genistein and coumestrol, are commonly used by many women as alternatives to HRT.

For this reason the effect of ovariectomy and estrogen replacement were studied on tumor formation in C57BL/6J-Min/+ (Min/+) mice, animals that bear a germline mutation in murine Apc. These mice develop multiple intestinal tumors that show loss of wild-type Apc protein. The results of this study suggest that endogenous estrogens protect against Apc-associated tumour formation and that tumour prevention by 17beta-estradiol is associated with an increase in ERbeta and a decrease in ERalpha expression in the target tissue(15, 16).

Again it clearly appears that ERbeta plays key role in the homeostasis of the intestinal epithelium.

The international patent application WO 01/77139 A1 describes 8β-substituted estratrienes wherein R⁸ means a straight-chain or branched-chain, optionally partially or completely halogenated alkyl or alkenyl radical with up to 5 carbon atoms, an ethinyl- or prop-1-inyl radical, as pharmaceutical active ingredients that have in vitro a higher affinity to estrogen receptor preparations of rat prostates than to estrogen receptor preparations of rat uteri, their production, their therapeutic use and pharmaceutical dispensing forms that contain the said compounds. Compounds with potent estrogen activity and a higher affinity to estrogen receptor preparations of rat prostates than to estrogen receptor preparations of rat uteri are ER- β selective ligands. The use of the above compounds for the prevention and therapy of neoplasias of the gastrointestinal tract is claimed in WO 01/77139 A1.

The compounds according to the present invention have been already described in WO 03/104253 A1, however their activity in the prevention and therapy of intestinal cancer, in particular adenoma and adenocarcinoma of the duodenum, ileum, colon, and rectum was not reported.

The document WO 2007/062877 A1 describes prodrugs of 9α-substituted estratrienes of the general formula (I) in which the group Z is bonded to the steroid, processes for their preparation, pharmaceutical compositions which comprise these compounds and use thereof. The compounds of the general formula I according to the invention do not bind directly to the estrogen receptor α and/or β. They bind to carboanhydrases and inhibit these enzymes.

Because colorectal cancer is a leading cause of cancer mortality in the industrialized world, a chemopreventive strategy and an effective treatment is of high importance.

### Disclosure of the invention

The present invention refers to the use of 9α-estra-1,3,5(10)-triene derivatives of formula (I) 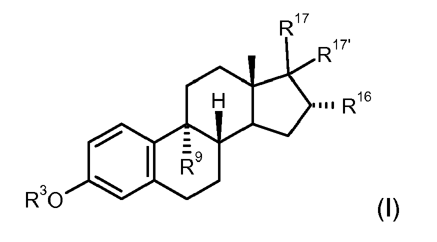 in which radicals R³, R⁹, R¹⁶, R¹⁷and R^{17'}, independently of one another, have the following meaning:
- R³: means a hydrogen atom or a group R¹⁸, in which
- R¹⁸: means a straight-chain or branched-chain, saturated or unsaturated C₁-C₆-alkyl radical, a trifluoromethyl group, an aryl, heteroaryl or aralkyl radical, a substituted aryl, heteroaryl radical with at least one radical independently chosen from a methyl, ethyl, trifluoromethyl, pentafluoroethyl, trifluoromethylthio, methoxy, ethoxy, nitro, cyano, halo- (fluorine, chlorine, bromine, iodine), hydroxy, amino, mono(C₁-C₈-alkyl) or di(C₁-C₈-alkyl)amino, whereby both alkyl groups are identical or different, di(aralkyl)amino, whereby both aralkyl groups are identical or different, carboxyl, carboxyalkoxy, C₁-C₂₀-acyl or C₁-C₂₀-acyloxy groups as substituents, an acyl radical -C(=O)R¹⁹, in which R¹⁹ is a straight-chain or branched-chain hydrocarbon radical with up to 10 carbon atoms that is saturated or unsaturated in up to three places and is partially or completely halogenated, or
- R¹⁸: means a group R²⁰SO₂, in which
- R²⁰: is an R²¹R²²N group, whereby R²¹ and R²², independently of one another, mean a hydrogen atom, a C₁-C₅-alkyl radical, a group C(O)R²³, in which R²³ means a unsubstituted or substituted, straight-chain or branched-chain hydrocarbon radical with up to 10 carbon atoms that is saturated or unsaturated in up to three places and is partially or completely halogenated, a cyclopropyl, cyclobutyl, cyclopentyl, cyclohexyl or cycloheptyl group, a C₄-C₁₅-cycloalkylalkyl radical with 3 to 7 carbon atoms in the cycloalkyl portion and with an alkyl portion of up to 8 carbon atoms or an aryl, heteroaryl or aralkyl radical, or a substituted aryl, or heteroaryl radical, with at least one radical independently chosen from a methyl, ethyl, trifluoromethyl, pentafluroethyl, trifluoromethylthio, methoxy, ethoxy, nitro, cyano, halo- (fluorine, chlorine, bromine, iodine), hydroxy, amino, mono(C₁-C₈-alkyl) or di(C₁-C₈-alkyl) amino, whereby both alkyl groups are identical or different, di (aralkyl)amino, whereby both aralkyl groups are identical or different, carboxyl, carboxyalkoxy, C₁-C₂₀-acyl or C₁-C₂₀-acyloxy groups as substituents, or, together with the N atom, a polymethylenimino radical with 4 to 6 C atoms or a morpholino radical,
- R⁹: is a straight-chain or branched-chain alkenyl or alkinyl radical with 2 to 6 carbon atoms, which optionally can be partially or completely fluorinated, or an ethinyl or prop-1-inyl radical,
- R¹⁶: means a hydroxy group or a group R¹⁸O-, R²⁰SO₂- or OC(O)R²³ with R¹⁸, R²⁰ and R²³ in each case in the meaning that is indicated under R³, or a para- or meta-sulphamoyl-benzoate radical optionally substituted with at least one radical independently chosen from a halogen atom, a nitrile group, a nitro group, a C₁-C₅-alkyl group, a CₚF₂ₚ₊₁ group with p = 1-3, a group OC(O)- C₁-C₅-alkyl, COOC₁-C₅-alkyl, OC₁₋-C₅- alkyl, C(O)NHC₁₋-C₅-alkyl or OC(O)NH-C₁₋-C₅-alkyl,
- R¹⁷ and R^{17'},: in each case independently of one another, are a hydrogen atom or a halogen atom

The present invention further refers to compounds of general formula I, in which R³ is a hydrogen atom.

According to a further embodiment of the invention the compounds of general formula I comprise the groups R⁹ , R¹⁶, R¹⁷ and R^{17'} in which R⁹ is a vinyl, ethinyl or prop-1-inyl group, R¹⁶ is a hydroxy group, R¹⁷ and R^{17'} are a hydrogen atom and a fluorine atom respectively or R¹⁷ and R^{17'} are both a hydrogen.

According to a particular form of realisation of the present invention R^{17'} is in β position.

Furthermore, compounds of general formula I, in which R¹⁶ stands for a group R¹⁸-O- or R¹⁹SO₂-O- with R¹⁸ and R¹⁹ in each case in the meaning that is indicated under R³ are also a further embodiment of the present invention.

Another embodiment of the present invention are the compounds according to general formula I in which R¹⁶ means a para- or meta-sulphamoyl-benzoate radical optionally substituted with at least one radical independently chosen from a hydrogen, a fluorine or chlorine atom, or a hydroxyl or a methoxy group.

In particular the present invention refers to the compounds:
9α-Vinyl-estra-1,3,5(10)-triene-3,16α-diol
9α-(2'-2'-Difluorovinyl)-estra-1,3,5(10)-triene-3,16α-diol
17β-Fluoro-9α-vinyl-estra-1,3,5(10)-triene-3,16α-diol
17,17 Difluoro-9α vinyl-estra-1,3,5(10)-triene-3,16α diol
3-Hydroxy-17β-fluoro-9α-vinylestra-1,3,5(10)-trien-16α-yl 3'-sulphamoylbenzoate
3-Hydroxy-9-vinylestra-1,3,5(10)-trien-16α-yl 3'-sulphamoylbenzoate
3-Hydroxy-17β-fluoro-9α-vinylestra-1,3,5(10)-trien-16α-yl-2'chlor -5'-sulphamoylbenzoate
3-Hydroxy-17β-fluoro-9α-vinylestra-1,3,5(10)-trien-16α-yl-4'-sulphamoylbenzoate

As halo- or halogen, a fluorine, chlorine, bromine or iodine atom is intended according to the present invention.

As alkyl radical is generally intended a (C₁-C₆)alkyl radical, as far as it is not differently specified, said alkyl radical being a straight or branched chain, saturated or unsaturated. Representative groups for an alkyl radical according to the present invention are methyl, ethyl, n- propyl, 1-methylethyl (iso-propyl), n-butyl, n-pentyl, 1,1-dimethylethyl (t-butyl) and n-hexyl. The C₁-C₆-alkyl radical can be partially or completely substituted by halogen atoms, hydroxy groups or C₁-C₆-alkoxy groups.

According to the above definition R¹⁸ is, for example, a methyl, ethyl, propyl, isopropyl, butyl, isobutyl, tert.-butyl, pentyl, isopentyl, neopentyl, or hexyl radical.

Alkoxy groups OR¹⁸ in the compounds of general formula I in each case can contain an alkyl radical according to the definition given above, whereby methoxy, ethoxy, propoxy, isopropoxy and t-butyloxy groups are preferred alkoxy radical.

Representatives of the C₁-C₅-alkyl radicals R²¹ and R²² are methyl, ethyl, propyl, isopropyl, butyl, isobutyl, tert-butyl, pentyl, isopentyl and neopentyl.

As representatives of straight-chain or branched-chain C₁-C₁₀ -alkyl radicals R²³, for example, methyl, ethyl, propyl, isopropyl, butyl, isobutyl, tert-butyl, pentyl, isopentyl, neopentyl, heptyl, hexyl, and decyl can be mentioned; methyl, ethyl, propyl and isopropyl are preferred.

As a C₃-C₇-cycloalkyl group, a cyclopropyl, cyclobutyl, cyclopentyl, cyclohexyl or cycloheptyl group can be mentioned.

A C₄-C₁₅-cycloalkylalkyl radical has 3 to 7 carbon atoms in the cycloalkyl portion; typical representatives are the cycloalkyl groups that are mentioned directly above. The alkyl portion has up to 8 carbon atoms.

As examples of a C₄-C₁₅-cycloalkylalkyl radical, the cyclopropylmethyl, cyclopropylethyl, cyclopentylmethyl, cyclopentylpropyl groups, etc., can be mentioned.

In terms of this invention, an aryl radical is a phenyl, 1- or 2-naphthyl, furyl-, thienyl, and pyridyl radical; the phenyl radical is preferred.

Examples of a heteroaryl radical according to the present invention are the 2-, 3- or 4-pyridinyl, the 2- or 3-furyl, the 2- or 3-thienyl, the 2-or 3-pyrrolyl, the 2-, 4- or 5-imidazolyl, the pyrazinyl, the 2-, 4- or 5-pyrimidinyl or 3- or 4-pyridazinyl radical.

As substituents that can be present on an aryl or heteroaryl radical, for example, a methyl-, ethyl-, trifluoromethyl-, pentafluoroethyl-, trifluoromethylthio-, methoxy-, ethoxy-, nitro-, cyano-, halogen- (fluorine, chlorine, bromine, iodine), hydroxy-, amino-, mono(C₁-C₈-alkyl) or di(C₁-C₈-alkyl)amino, whereby both alkyl groups are identical or different, di(aralkyl)amino, whereby both aralkyl groups are identical or different, carboxyl, carboxyalkoxy, C₁-C₂₀-acyl or C₁-C₂₀-acyloxy groups can be mentioned.

An aralkyl radical is a radical that contains in the ring up to 14, preferably 6 to 10 C atoms, and in the alkyl chain 1 to 8, preferably 1 to 4, C atoms. Thus, as aralkyl radicals, for example, benzyl, phenylethyl, naphthylmethyl, naphthylethyl, furylmethyl, thienylethyl, and pyridylpropyl are suitable.

A vinyl or allyl radical is primarily defined with a C₂-C₆-alkenyl radical.

A C₂-C₆₋alkinyl radical is preferably defined as an ethinyl radical or a prop-1-inyl radical.

C₁-C₁₀-Acyl radicals mean, for example, acetyl, propionyl, butyryl, valeroyl, isovaleroyl, pivaloyl, hexanoyl, octyl, nonyl, or decanoyl.

One or two hydroxyl groups at C atoms 3 and 16 can be esterified with an aliphatic, straight-chain or branched-chain, saturated or unsaturated C₁-C₁₄-mono- or polycarboxylic acid or an aromatic carboxylic acid.

Suitable as such carboxylic acids for esterification are, for example:
- Monocarboxylic acids: formic acid, acetic acid, propionic acid, butyric acid, isobutyric acid, valeric acid, isovaleric acid, pivalic acid, lauric acid, myristic acid, acrylic acid, propionic acid, methacrylic acid, crotonic acid, isocrotonic acid, oleic acid, and elaidic acid. Esterification with acetic acid, valeric acid or pivalic acid is preferred.
- Dicarboxylic acids: oxalic acid, malonic acid, succinic acid, glutaric acid, adipic acid, pimelic acid, suberic acid, azelaic acid, sebacic acid, maleic acid, fumaric acid, muconic acid, citraconic acid, and mesaconic acid.
- Aromatic carboxylic acids: benzoic acid, phthalic acid, isophthalic acid, terephthalic acid, naphthoic acid, o-, m- and p-toluic acid, hydratropic acid, atropic acid, cinnamic acid, nicotinic acid, and isonicotinic acid.
Esterification with benzoic acid is preferred.

As prodrugs, the esters of the 9α-substituted estratrienes according to the invention have advantages compared to the unesterified active ingredients with respect to their method of administration, their type of action, strength and duration of action.

The compounds according to the present invention are particularly active as ER-β agonists.

Especially the sulfamates of 9α-substituted estratrienes according to the invention have pharmacokinetic and pharmacodynamic advantages. Related effects were already described in other steroid-sulfamates (J. Steroid Biochem. Molec. Biol, 55, 395-403 (1995); Exp. Opinion Invest. Drugs 7, 575-589 (1998)).

The amount of a compound of general formula I, as specific Estrogen Receptor β (ERβ ) ligand for the prevention and treatment of intestinal cancer, in particular of duodenal or colorectal adenoma and adenocarcinoma that is to be administered varies within a wide range and can cover any effective amount.

On the basis of the condition that is to be treated and the type of administration, the amount of the compound that is administered can be 0.01 µg/kg to 100 mg/kg of body weight, preferably 0.04 µg/kg to 1 mg/kg of body weight, per day. In humans, this corresponds to a dose of 0.8 µg to 8 g, preferably 3.2 µg to 80 mg, daily. According to the invention, a dosage unit contains 1.6 µg to 2000 mg of one or more compounds of general formula I.

The compounds according to the invention and the acid addition salts are suitable for the production of pharmaceutical compositions and preparations. The pharmaceutical compositions or pharmaceutical agents contain as active ingredients one or more of the compounds according to the invention or their acid addition salts, optionally mixed with other pharmacologically or pharmaceutically active substances. The production of the pharmaceutical agents is carried out in a known way, where the known and commonly used pharmaceutical adjuvants as well as other commonly used vehicles and diluents can be used.

As such vehicles and adjuvants, for example, those are suitable that are recommended or indicated in the following bibliographic references as adjuvants for pharmaceutics, cosmetics and related fields: Ullmans Encyklopädie der technischen Chemie [Ullman's Encyclopedia of Industrial Chemistry], Volume 4 (1953), pages 1 to 39; Journal of Pharmaceutical Sciences, Volume 52 (1963), page 918 ff., issued by Czetsch-Lindenwald, Hilfsstoffe für Pharmazie und angrenzende Gebiete [Adjuvants for Pharmaceutics and Related Fields]; Pharm. Ind., Issue 2, 1961, p. 72 and ff.: Dr. H.P. Fiedler, Lexikon der Hilfsstoffe für Pharmazie, Kosmetik und angrenzende Gebiete [Dictionary of Adjuvants for Pharmaceutics, Cosmetics and Related Fields], Cantor KG, Aulendorf in Württemberg 1971.

The compounds can be administered orally or parenterally, for example intraperitoneally, intramuscularly, subcutaneously or percutaneously. The compounds can also be implanted in the tissue.

For oral administration, capsules, pills, tablets, coated tablets, etc., are suitable. In addition to the active ingredient, the dosage units can contain a pharmaceutically compatible vehicle, such as, for example, starch, sugar, sorbitol, gelatin, lubricant, silicic acid, talc, etc.

For parenteral administration, the active ingredients can be dissolved or suspended in a physiologically compatible diluent. As diluents, very often oils with or without the addition of a solubilizer, a surfactant, a suspending agent or an emulsifying agent are used. Examples of oils that are used are olive oil, peanut oil, cottonseed oil, soybean oil, castor oil and sesame oil.

The compounds can also be used in the form of a depot injection or an implant preparation, which can be formulated so that a delayed release of active ingredient is made possible.

As inert materials, implants can contain, for example, biodegradable polymers, or synthetic silicones such as, for example, silicone rubber. In addition, for percutaneous administration, the active ingredients can be added to, for example, a patch.

For the production of intravaginal systems (e.g., vaginal rings) or intrauterine systems (e.g., pessaries, coils, IUDs, Mirena(R)) that are loaded with active compounds of general formula I for local administration, various polymers are suitable, such as, for example, silicone polymers, ethylene vinyl acetate, polyethylene or polypropylene.

To achieve better bio-availability of the active ingredient, the compounds can also be formulated as cyclodextrin clathrates. For this purpose, the compounds are reacted with α-, β-, or γ-cyclodextrin or derivatives of the latter (PCT/EP95/02656).

According to the invention, the compounds of general formula I can also be encapsulated with liposomes.

### Brief description of the drawings

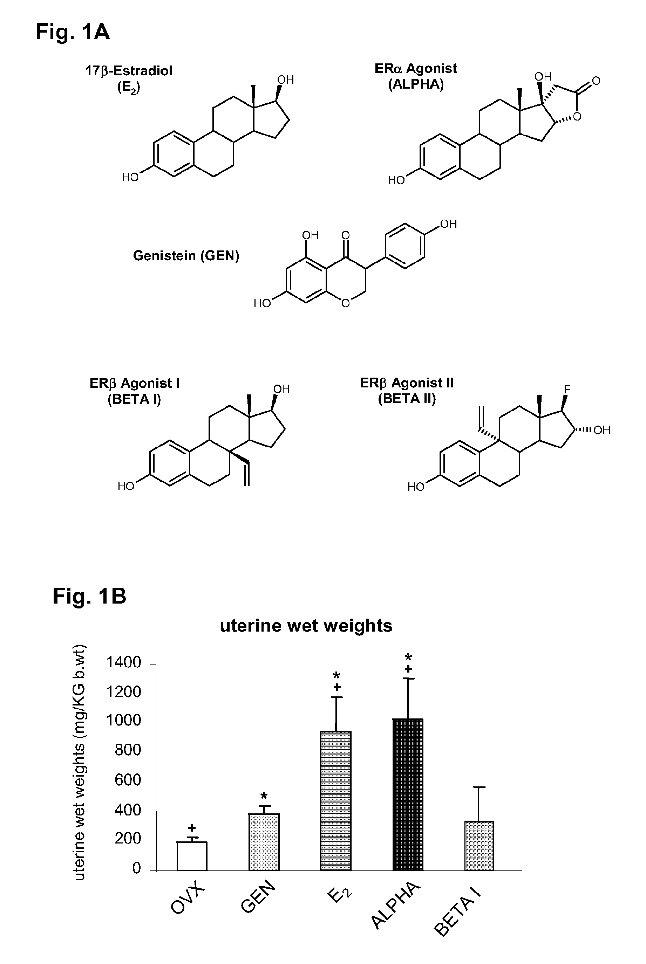

### Fig. 1A: Selected compounds and estrogenic potency - ER selective agonists

Chemical structures of Genistein (Gen), 17β-estradiol (E2) and the specific ER ligands with an agonistic effect: 3,17-dihydroxy-19-nor-17α-pregna-1,3,5(10)-triene-21,16 α-lactone (ALPHA), 8β-vinyl-estra-1,3,5(10)-triene-3,17β-diol (BETA I) and 17β-Fluoro-9α-vinyl-estra-1,3,5(10)-triene-3,16α-diol (BETA II).

### Fig. 1B: Selected compounds and estrogenic potency - Uterine wet weights

Uterine wet weights after 3 weeks of treatment with E₂, ALPHA, BETA I, BETA I+ICI and GEN. OVX= ovariectomized control group. Experimental conditions and treatment procedures are given in Materials and Methods.
(*) denotes values significantly different from ovariectomized group (OVX) P≤ 0.01, ANOVA, n=6

### Fig. 2: Immunohistochemical staining of PCNA in ileal mucosa cells

Depicted are cross sections of the ileum of OVX rats after immunohistochemical staining of PCNA-positive nuclei.
Experimental conditions and treatment procedures are given in Materials and Methods. Magnification: 200x.

### Fig. 3A: Western Blot analysis of markers for proliferation and apoptosis

Depicted are representative Western Blots and the densitometric evaluation of PCNA and active caspase-3. Actin serves as reference. Experimental conditions and treatment procedures are given in Materials and Methods.

### Fig. 3B: Effects of E2, Gen, BETA I and ALPHA on the PCNA mRNA Expression in the intestinal Mucosa.

PCNA mRNA Expression in the intestinal Mucosa is measured. Experimental conditions and treatment procedures are given in Materials and Methods.
The diminished PCNA mRNA Expression in the testing group Gen and BETA I is clearly recognisable.

### Fig. 4: Evidence of the antagonising effect of BETA I

Western Blots and densitometric evaluation of PCNA and active caspase-3 protein expression in pooled ileal tissue are presented. Actin serves as reference. Experimental conditions and treatment procedures are given in Materials and Methods.

### Fig. 5: Doses effect BETA II on the intestinal mucosa.

BETA II was tested at 5, 15 and 50 µg kg-1 b.wt d-1 to determine the doses effect in the intestinal mucosa. Experimental conditions and treatment procedures are given in Materials and Methods.

### Fig. 6. Effects of BETA II compared to BETA I.

Depicted are representative Western Blots and the densitometric evaluation of PCNA and active caspase-3 protein expression in pooled ileal tissue. Actin serves as reference. Experimental conditions and treatment procedures are given in Materials and Methods.

Abbreviations: BETA I treated group at a 5µg dosis, BETA II treated group at a 5µg dosis, OVX= ovariectomized control group.

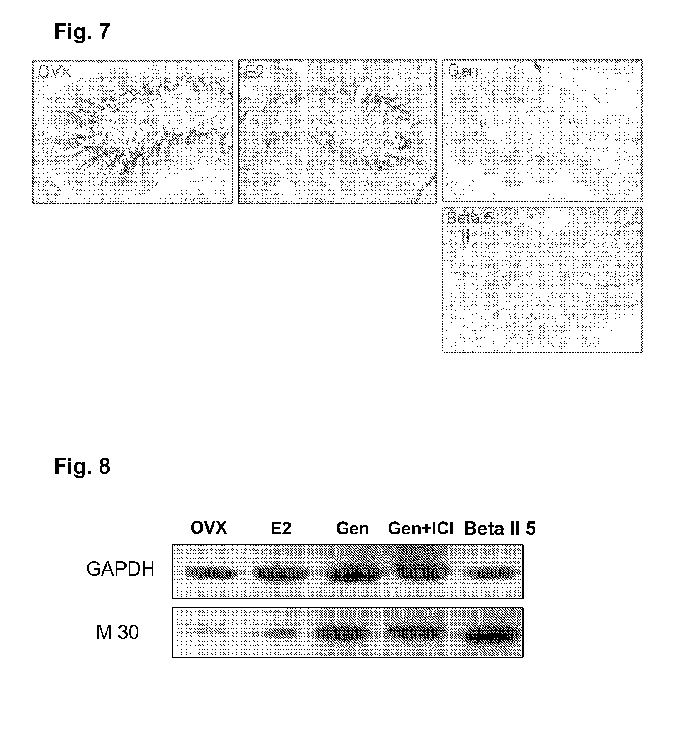

### Fig. 7 Immunohistochemical staining of PCNA in colonic mucosa cells of E2, Gen, BETA II at 5µg on apoptosis in Colon

Depicted are cross sections of the colon of OVX rats after immunohistochemical staining of PCNA-positive nuclei and the quantitative evaluation of the staining results. Eight crypts of each animal were counted and statistically averaged. Experimental conditions and treatment procedures are given in Materials and Methods.
Magnification: 50x.

### Fig. 8. Effects of E2, Gen and BETA II at 5µg on apoptosis in Colon.

BETA II causes already at a dose of 5µg a significant stimulation of the Cytokeratin 18 cleavage (M30).

### Mode(s) for carrying out the invention

In support to the present invention several experimental activities were performed. First of all the effect on the uterus of ovariectomized animals treated with E₂, ALPHA, BETA I, and GEN was analyzed.

Treatment of OVX rats with E₂ led to a strong stimulation of uterine wet weights, whereas application of GEN resulted only in a faint, although significant increase (Fig. 1B). Application of ALPHA strongly increased uterine wet weights, while BETA I treated animals showed no significant uterus stimulation (Fig. 1B).

To investigate effects of the different estrogenic compounds on intestinal tissue homeostasis, protein expression of the proliferation marker PCNA was determined by immunohistochemistry and Western Blot analysis.

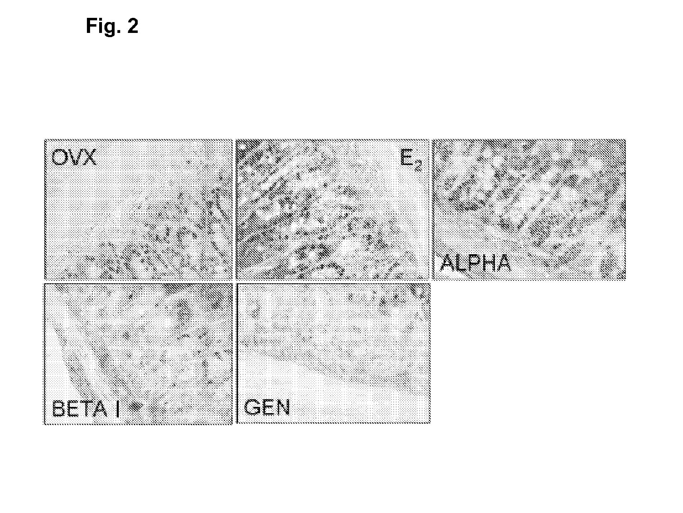

Immunostaining of ileal cross sections revealed that in this area of the digestive tract, PCNA protein expression in OVX animals was slightly induced by administration of E₂ and ALPHA (Fig.2).

Interestingly, treatment of OVX animals with BETA I and GEN significantly reduced PCNA protein expression in ileal tissue sections (Fig.2).

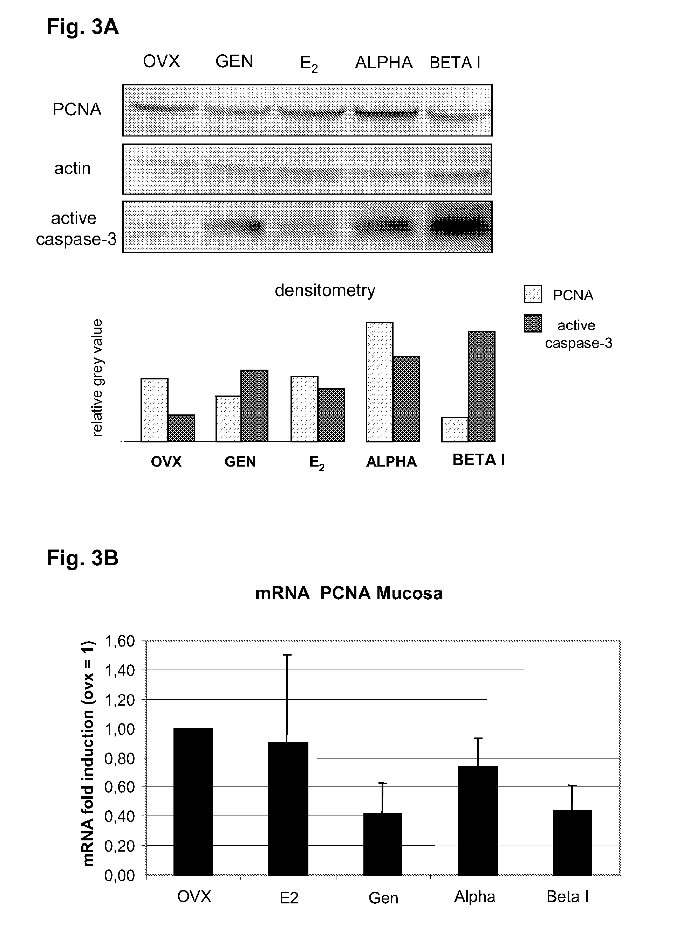

This observation was supported by Western Blot analysis of ileal mucosa cells (Fig.3).

The densitometric evaluation revealed that PCNA expression of intestinal mucosa cells of OVX rats was not affected by application of E₂, while treatment with ALPHA lead to an elevation of PCNA expression (Fig.3A-B). In contrast, the treatment of OVX animals with BETA I and GEN reduced the expression patterns of this marker for proliferation (Fig.3A-B).

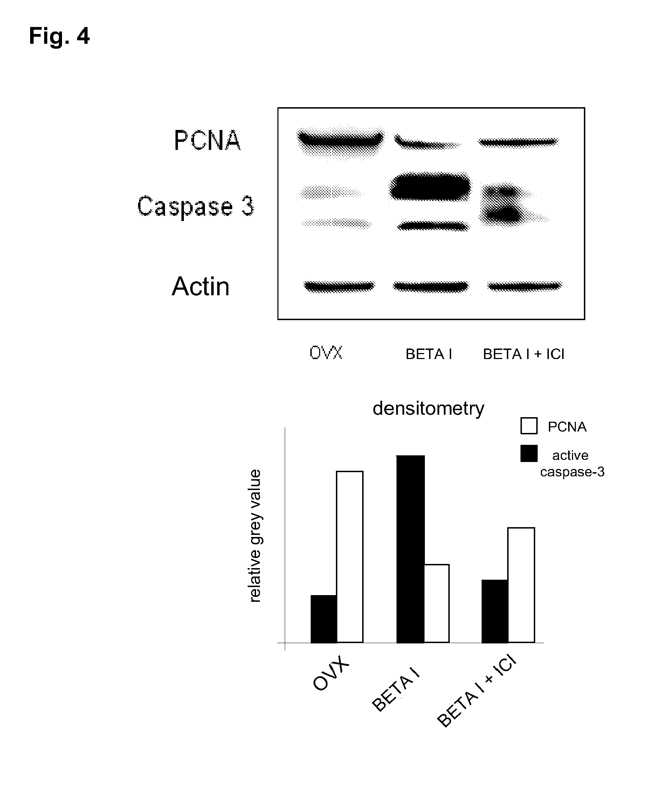

In addition, activation of caspase-3 was dramatically promoted by application of BETA I and moderately by GEN and ALPHA treatment, while activation of this apoptotic key enzyme remained on a low level in OVX and E₂ treated animals (Fig.3). Co-administration of ICI resulted in an antogonization of pro-apoptotic and anti-proliferative effects of BETA I (Fig. 4A-B).

The results of the tests mentioned above indicate that the administration of the ERbeta selective agonist BETA I inhibits the cell proliferation in the intestinal epithelium and increases the apoptotic rate. This clearly results in a decrease of the tissue cell number which is an essential parameter in prevention and treatment of cancer.

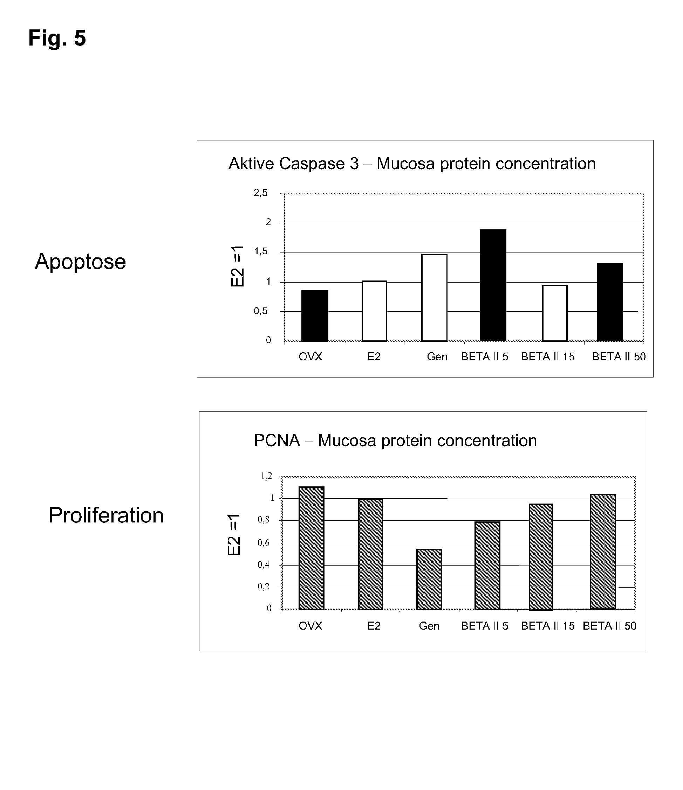

In a subsequent test , the potency of 17β-Fluoro-9α-vinyl-estra-1,3,5(10)-triene-3,16α-diol (BETA II), a compound according to the invention, is determined in relation to the described effect under the same experimental design (Fig. 5-6).

It was surprisingly found that BETA II has its strongest effect at lowest tested dose of 5µg/kg. At said dose the compound according to the invention does not show any measurable effect on the uterus weight and has an activity which is about 20 times higher than BETA I in relation to the to effects regulating the intestinal homeostasis as discussed above (Fig. 5).

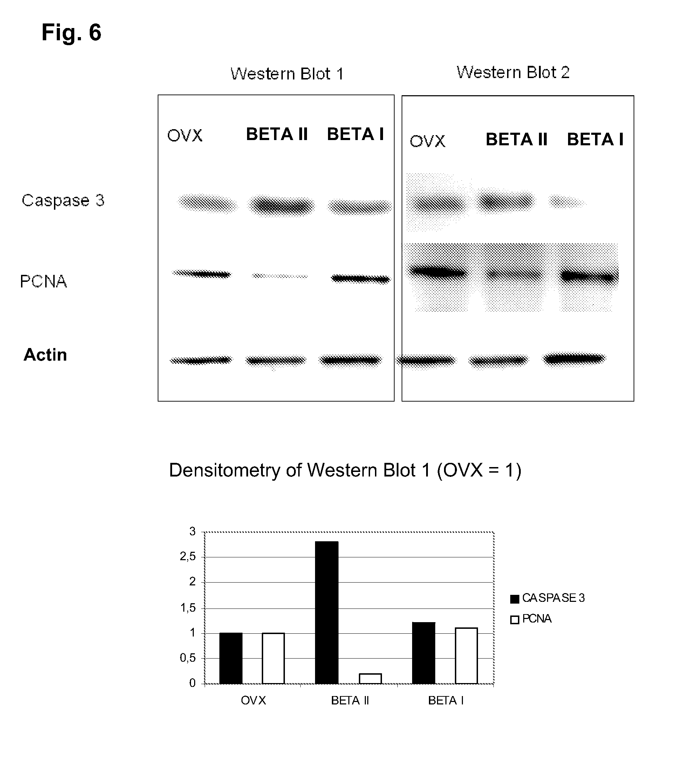

The favourable profile of the compounds according to the present invention, which clearly promote the activation of the caspase-3 at a higher level than the effect achieve with compounds already known in the art, is confirmed when testing BETA I and BETA II at the same concentration (Fig. 6). The improved effect achieved with BETA II is evident also with reference to down regulation of the expression of the proliferation marker PCNA.

The activation of the caspase-3 on one side and the contemporary down regulation of the PCNA on the other side reveal a clear synergistic action of the ERβ selective ligands according to the invention. Such a favourable synergistic effect on the chosen markers clearly appear from the delta represented in the diagram of Fig. 6 between the grey scale level of the caspase and PCNA with reference to BETA II.

Further tests are performed, where ER-specific effects of E2, GEN and BETA II, according to the invention, are examined on normal rectal colonic tissue homeostasis. As part of this investigation, PCNA protein expression in rectal colonic cross sections is analyzed by immunohistochemistry. Furthermore, an M 30 antibody that is known to be a useful tool for the assessment of apoptosis in colorectal tissues (17) is used in Western Blot analysis (Fig. 8).

In contrast to E₂, and according to its effect on the small intestine, GEN but specifically the compound according to the present invention BETA II reduced PCNA expression in colonic mucosa cells already at a dose of 5µg/Kg (Fig.7).

As depicted in figure 8, application of BETA II, according to the invention, dramatically induced cleavage of cytokeratin 18 (M 30) in colonic mucosa cells of OVX rats, whereas the amount of cleaved cytokeratin 18 in epithelial colonic cells was only marginally increased after E2 treatment

The results as presented in figures 7 and 8 further demonstrate the anti-prolifarative and pro-apoptotic effects of the compounds according to the invention also on the homeostasis of the colon epithelium. Such effect are essential for prevention and treatment of intestinal cancer in particular adenoma and adenocarcinoma of the duodenum, ileum, colon, and rectum.

### Materials and Methods

### Substances

17β-Estradiol (Estra-1,3,5(10)-trien-3,16α,17 β-diol) E₂, genistein (4',5,7-trihydroxyisoflavone) Gen, specific ER-α agonist (3,17-dihydroxy-19-nor-17α-pregna-1,3,5 (10)-triene-21,16α-lactone) ALPHA, specific ER- β agonists (8-vinylestra-1,3,5 (10)-triene-3,17β-diol) BETA I and 8β-vinylestra-1,3,5(10)-triene-3,17β-diol (BETA I) and 17β-Fluoro-9α-vinyl-estra-1,3,5(10)-triene-3,16α-diol (BETA II), Faslodex (ICI)

### Diet

All rats had free access to a diet low in phytoestrogen content (ILD) (SSniff GmbH, Soest, Germany) and water ad libitum.

### Animals

Adult female Wistar rats (200-220g) were obtained from Janvier (Janvier, Le Genest St Isle, France) and were maintained under controlled conditions of temperature (20°C ± 1, relative humidity 50-80%) and illumination (12 h light, 12 h dark). All animal experiments were approved by the Committee on Animal Care and complied with accepted veterinary medical practice.

### Animal treatment and tissue preparation

Post pubertal animals were ovariectomized (OVX) at the age of three months and weighing 200-220g. After 14 days of endogenous hormonal decline the animals were treated with the test compounds or vehicle for three weeks. The animals were randomly allocated to treatment and vehicle groups (n = 6). E2 (4 µg kg-1 b.wt d-1), GEN (10 mg kg-1 b.wt d-1), ALPHA (10 µg kg-1 b.wt d-1), BETA I (100 µg kg-1 b.wt d-1), BETA II (5-15-50 µg kg-1 b.wt d-1) and ICI (3 mg kg-1 b.wt d-1) were dissolved in dimethylsulfoxide (DMSO) (200 µl/KG b. wt/d) and corn oil (800 µl/KG b. wt/d) for s.c. administration.

The treatment doses of the respective substances were chosen based on previous experiments (18). GEN has been demonstrated to be effective at a dose of 10 mg/kg/d (19; 20). For isotype-specific ER activation, we used the selective ER-agonists ALPHA and BETA I (Fig.1A). Because these compounds activate both receptors at higher concentrations, doses of 10µg kg-1 b.wt d-1 (ALPHA) and 100 µg kg-1 b.wt d-1 (BETA I) respectively were chosen. For these doses, the action through either ERα or ERβ respectively can be anticipated (21; 22).

In order to antagonize ER-specific effects of BETA I, OVX animals were co-treated with BETA I (100 µg kg-1 b.wt d-1) and ICI (3 mg kg-1 b.wt d-1).

BETA II was tested at 5,15 and 50 µg kg-1 b.wt d-1.

Animals were sacrificed by decapitation after light anesthesia with CO₂ inhalation. Uteri and intestinal tissues were prepared free of fat and were fixed in 4% formalin (for immunohistochemical analysis) and liquid nitrogen (for molecular biology). The uterine wet weights were determined. Western Blot analysis

Ileal mucosa and rectal colon were dissected from the animals and immediately stored in liquid nitrogen. Pooled frozen tissue (n= 6 animals per group) was powdered and homogenized in a buffer (623.5 nM Tris pH 8 EDTA) containing enzyme inhibitors (5 mg/ml aprotonin, 5 mg/ml leupeptin, 1 mg/ml pepstatin-A, 5 mg/ml antipain, 100 mM pefac in 0.5 M EDTA, pH 8). Protein concentrations were determined by the method of Lowry (Dc Protein Assay, Bio-Rad). Equal amounts of sample (40 µg protein) were loaded on a Protean II ready Gel (Bio-Rad).

After electrophoresis and separation, samples were transferred onto nitrocellulose membranes and blocked with 5% BSA in Phosphate (100 mM)-buffered saline solution (pH 7.4) at room temperature for 2 hours. The immobilized proteins were quantitatively detected using specific antibodies for actin and GAPDH (A5060 and G8795, Sigma-Aldrich, Steinheim, Germany), PCNA Clone PC 10 (Dako, Glostrup, Denmark), M30 Cytodeath (ALX-804-590, Alexis, San Diego, CA, USA) and active caspase-3 (557035, BD Pharmingen, Heidelberg, Germany).

Polyclonal Swine Anti-Rabbit Immunglobulins/HRP and Polyclonal Rabbit Anti-Mouse Immunglobulins/HRP (P0217 and P0260, Dako, Glostrup, Denmark) were used as species-specific HRP-conjugated secondary antibodies.

Blot signals were visualized by the chemoluminescent POD-Substrate (Lumi-Light Plus, Roche Diagnostics, Mannheim, Germany) and a Fluorchem Luminescent Imager (Alpha Innotech, CA, USA).

The protein bands were quantified by densitometry using ImageJ 1.38. To account for in homogenous protein loading of the slots we calculated the ratio of the reference protein (Actin, GAPDH) to the target protein (PCNA, M30, active caspase-3).

### Immunohistochemistry

Formalin-fixed intestinal samples were embedded in paraffin, sliced with a microtome (7µm sections). After deparaffinization, antigen retrieval was performed in an incubator for 20 h at 60°C in 0,01 M citrate buffer pH 6,0.

Subsequently, endogenous peroxidases were blocked for 20 min. with 3% hydrogen peroxide and the slides were covered with a 0,25% Triton X-100 solution for 10 min.

The primary antibody, PCNA Clone PC10 (Dako, Glostrup, Denmark) was applied at dilutions of 1:100, respectively.

After 1 h incubation with the primary antibodies, samples were incubated with the secondary antibody: Polyclonal Goat Anti-Mouse Immunglobulins/Biotinylated (Dako, Glostrup, Denmark).

Afterwards the sections were covered with Streptavidin-biotinylated Horseradish Peroxidase Complex (GE Healthcare, Little Chalfont Buckinghamshire, UK) at a dilution of 1:400. The samples were developed with DAB for 10 min. For negative controls, slides were immunostained in the absence of the primary antibody.

### Evaluation of staining results

Quantitative analysis was performed in complete crypts of normal mucosa. Brown nuclear staining was indicative of a positive PCNA signal.

### Statistical analysis

All data are expressed as arithmetic means with their standard errors. Statistical significance of differences was calculated using one-way analysis of variance (ANOVA) with following Tukey HSD test where it was appropriate. Statistical tests were used for comparisons between every two groups and evaluated using P< 0.01.

Booth C, Hargreaves DF, O'Shea JA, Potten CS (1999). In vivo administration of genistein has no effect on small intestinal epithelial proliferation and apoptosis, but a modest effect on clonogen survival. Cancer Lett 144(2):169-75.

### References

1. Nelson HD, Humphrey LL, Nygren P, Teutsch SM, Allan JD (2002). Postmenopausal hormone replacement therapy: scientific review. JAMA 288(7):872-81 Review.
2. Rossouw,J., Anderson,G., Prentice,R. et al. (2002) Risks and benefits of estrogen and progesterone in healthy post-menopausal women: principal results from the Women's Health Initiative randomized controlled trial. J. Am. Med. Assoc., 288, 321--333
3. Wada-Hiraike O, Imamov O, Hiraike H, Hultenby K, Schwend T, Omoto Y, Warner M, Gustafsson JA (2006a). Role of estrogen receptor beta in colonic epithelium. Proc Natl Acad Sc USA;103(8):2959-64.
4. Chlebowski RT, Wactawski-Wende J, Ritenbaugh C, Hubbell FA, Ascensao J, Rodabough RJ, Rosenberg CA, Taylor VM, Harris R, Chen C, Adams-Campbell LL, White E; Women's Health Initiative Investigators (2004). Estrogen plus progestin and colorectal cancer in postmenopausal women. N Engl J Med 350(10):991-1004.
5. Farquhar CM, Marjoribanks J, Lethaby A, Lamberts Q, Suckling JA, Cochrane HT Study Group (2005). Long term hormone therapy for perimenopausal and postmenopausal women. Cochrane Database Syst Rev (3):CD004143 Review.
6. Dinger JC, Heinemann LA, Möhner S, Thai do M, Assmann A (2007). Colon cancer risk and different HRT formulations: a case-control study. BMC Cancer 7:76.
7. Carpenter KD, Korach KS (2006). Potential biological functions emerging from the different estrogen receptors. Ann N Y Acad Sci 1092:361-73 Review.
8. Campbell-Thompson M, Lynch IJ, Bhardwaj B (2001). Expression of estrogen receptor (ER) subtypes and ERbeta isoforms in colon cancer. Cancer Res 15; 61 (2):632-40.
9. Konstantinopoulos PA, Kominea A, Vandoros G, Sykiotis GP, Andricopoulos P, Varakis I, Sotiropoulou-Bonikou G, Papavassiliou AG (2003). Oestrogen receptor beta (ERbeta) is abundantly expressed in normal colonic mucosa, but declines in colon adenocarcinoma paralleling the tumour's dedifferentiation. Eur J Cancer 39(9):1251-8.
10. Xie LQ, Yu JP, Luo HS (2004). Expression of estrogen receptor beta in human colorectal cancer. World J Gastroenterol 10(2):214-7.
11. Martineti V, Picariello L, Tognarini I, Carbonell Sala S, Gozzini A, Azzari C, Mavilia C, Tanini A, Falchetti A, Fiorelli G, Tonelli F, Brandi ML (2005). ERbeta is a potent inhibitor of cell proliferation in the HCT8 human colon cancer cell line through regulation of cell cycle components. Endocr Relat Cancer 12(2):455-69.
12. Jassam N, Bell SM, Speirs V, Quirke P (2005). Loss of expression of oestrogen receptor beta in colon cancer and its association with Dukes' staging. Oncol Rep 14(1):17-21.
13. Wada-Hiraike O, Warner M, Gustafsson JA (2006b). New developments in oestrogen signalling in colonic epithelium. Biochem Soc Trans 34(Pt 6):1114-6.
14. Lechner D, Kallay E, Cross HS (2005). Phytoestrogens and colorectal cancer prevention. Vitam Horm 70:169-98 Review.
15. Bertagnoli M et al.Cancer Res. 2001 Mar 15;61(6):2547-51.
16. Bertagnoli M et al. Carcinogenesis. (2005);26(3):587-95.
17. Koornstra JJ, Rijcken FE, De Jong S, Hollema H, de Vries EG, Kleibeuker JH. Assessment of apoptosis by M30 immunoreactivity and the correlation with morphological criteria in normal colorectal mucosa, adenomas and carcinomas, Histopathology 2004, 44(1):9-17.
18. Hertrampf T, Seibel J, Laudenbach U, Fritzemeier KH, Diel P (2008). Analysis of the effects of oestrogen receptor alpha (ERalpha)- and ERbeta-selective ligands given in combination to ovariectomized rats. Br J Pharmacol. 153(7):1432-7.
19. Diel P, Geis RB, Caldarelli A, Schmidt S, Leschowsky UL, Voss A, Vollmer G (2004). The differential ability of the phytoestrogen genistein and of estradiol to induce uterine weight and proliferation in the rat is associated with a substance specific modulation of uterine gene expression. Mol and Cell Endocrinol 30;221(1-2), 21-32.
20. Hertrampf T, Schmidt S, Laudenbach-Leschowsky U, Seibel J, Diel P (2005). Tissue-specific modulation of cyclooxygenase-2 (Cox-2) expression in the uterus and the v. cava by estrogens and phytoestrogens. Mol Cell Endocrinol 243(1-2):51-7.
21. Hegele-Hartung C, Siebel P, Peters O, Kosemund D, Muller G, Hillisch A, Walter A, Kraetzschmar J, Fritzemeier KH (2004). Impact of isotype-selective estrogen receptor agonists on ovarian function. Proc of the Natl Acad Sci USA 101(14), 5129-34.
22. Hillisch A, Peters O, Kosemund D, Muller G, Walter A, Schneider B, Reddersen G, Elger W, Fritzemeier KH (2004). Dissecting physiological roles of estrogen receptor alpha and beta with potent selective ligands from structure-based design. Mol Endocrinol 18(7), 1599-609.

## Claims

**1.** Use of 9α-estra-1,3,5(10)-triene derivatives of formula (I) 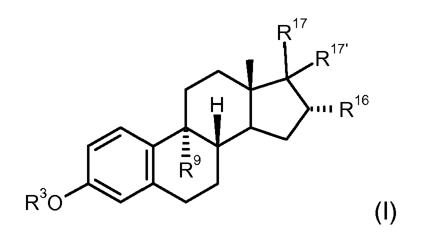 in which radicals R³, R⁹, R¹⁶, R¹⁷and R^{17'}, independently of one another, have the following meaning:
R³ means a hydrogen atom or a group R¹⁸, in which
R¹⁸ means a straight-chain or branched-chain, saturated or unsaturated C₁-C₆-alkyl radical, a trifluoromethyl group, an aryl, heteroaryl or aralkyl radical, a substituted aryl, heteroaryl radical with at least one radical independently chosen from a methyl, ethyl, trifluoromethyl, pentafluoroethyl, trifluoromethylthio, methoxy, ethoxy, nitro, cyano, halo- (fluorine, chlorine, bromine, iodine), hydroxy, amino, mono(C₁₋₈-alkyl) or di(C₁₋₈-alkyl)amino, whereby both alkyl groups are identical or different, di(aralkyl)amino, whereby both aralkyl groups are identical or different, carboxyl, carboxyalkoxy, C₁-C₂₀-acyl or C₁-C₂₀-acyloxy groups as substituents, an acyl radical -C(=O)R¹⁹, in which R¹⁹ is a straight-chain or branched-chain hydrocarbon radical with up to 10 carbon atoms that is saturated or unsaturated in up to three places and is partially or completely halogenated, or
R¹⁸ means a group R²⁰SO₂, in which R²⁰ is an R²¹R²²N group, whereby R²¹ and R²², independently of one another, mean a hydrogen atom, a C₁-C₅-alkyl radical, a group C(O)R²³, in which R²³ means a unsubstituted or substituted, straight-chain or branched-chain hydrocarbon radical with up to 10 carbon atoms that is saturated or unsaturated in up to three places and is partially or completely halogenated, a cyclopropyl, cyclobutyl, cyclopentyl, cyclohexyl or cycloheptyl group, a C₄-C₁₅-cycloalkylalkyl radical with 3 to 7 carbon atoms in the cycloalkyl portion and with an alkyl portion of up to 8 carbon atoms or an aryl, heteroaryl or aralkyl radical, or a substituted aryl, or heteroaryl radical, with at least one radical independently chosen from a methyl, ethyl, trifluoromethyl, pentafluroethyl, trifluoromethylthio, methoxy, ethoxy, nitro, cyano, halo- (fluorine, chlorine, bromine, iodine), hydroxy, amino, mono(C₁-C₈-alkyl) or di(C₁-C₈-alkyl) amino, whereby both alkyl groups are identical or different, di (aralkyl)amino, whereby both aralkyl groups are identical or different, carboxyl, carboxyalkoxy, C₁ -C₂₀-acyl or C₁ -C₂₀-acyloxy groups as substituents, or, together with the N atom, a polymethylenimino radical with 4 to 6 C atoms or a morpholino radical,
R⁹ is a straight-chain or branched-chain alkenyl or alkinyl radical with 2 to 6 carbon atoms, which optionally can be partially or completely fluorinated, or an ethinyl or prop-1-inyl radical,
R¹⁶ means a hydroxy group or a group R¹⁸O-, R²⁰SO₂- or OC(O)R²³ with R¹⁸, R²⁰ and R²³ in each case in the meaning that is indicated under R³, or a para- or meta-sulphamoyl-benzoate radical optionally substituted with at least one radical independently chosen from a halogen atom, a nitrile group, a nitro group, a C₁₋₅-alkyl group, a CₚF₂ₚ₊₁ group with p = 1-3, a group OC(O)- C₁₋₅-alkyl, COOC₁₋₅-alkyl, OC₁₋₅-alkyl, C(O)NHC₁₋₅-alkyl or OC(O)NH-C₁₋₅-alkyl,
R¹⁷ and R^{17'}, in each case independently of one another, are a hydrogen atom or a halogen atom
as specific Estrogen Receptor β (ERβ ) ligand for the prevention and treatment of intestinal cancer.

**2.** A 9α-estra-1,3,5(10)-triene derivatives of formula (I) according to claim 1 in which R³ is a hydrogen atom.

**3.** A 9α-estra-1,3,5(10)-triene derivatives of formula (I) according to claim 1 in which R⁹ is a vinyl, ethinyl or prop-1-inyl group, R¹⁶ is a hydroxy group, R¹⁷ and R^{17'} are a hydrogen atom and a fluorine atom respectively, or R¹⁷ and R^{17'} are both a hydrogen.

**4.** A 9α-estra-1,3,5(10)-triene derivatives of formula (I) according to claim 1 or 3 in which R¹⁶ means a para- or meta-sulphamoyl-benzoate radical optionally substituted with at least one radical independently chosen from a hydrogen, a fluorine or chlorine atom, or a hydroxyl or a methoxy group.

**6.** A 9α-estra-1,3,5(10)-triene derivatives of formula (I) according to claim 1 with the following chemical formula:
9α-Vinyloestra-1,3,5(10)-triene-3,16α-diol
9α-(2'-2'-Difluorovinyl)-oestra-1,3,5(10)-triene-3,16α-diol
17β-Fluoro-9α-vinyl-oestra-1,3,5(10)-triene-3,16α-diol
17,17 Difluoro-9α vinyl-oestra-1,3,5(10)-triene-3,16α diol
3-Hydroxy-17β-fluoro-9α-vinyloestra-1,3,5(10)-trien-16α-yl 3'-sulphamoylbenzoate
3-Hydroxy-9-vinyloestra-1,3,5(10)-trien-16α-yl 3'-sulphamoylbenzoate
3-Hydroxy-17β-fluoro-9α-vinyloestra-1,3,5(10)-trien-16α-yl-2'chlor -5-sulphamoylbenzoate
3-Hydroxy-17β-fluoro-9α-vinyloestra-1,3,5(10)-trien-16α-yl-4'-sulphamoylbenzoate

**7.** A 9α-estra-1,3,5(10)-triene derivatives of formula (I) according to any one of the claims 1to 6 for the prevention and treatment of adenoma of the duodenum, ileum, colon, and rectum.

**8.** A 9α-estra-1,3,5(10)-triene derivatives of formula (I) according to any one of the claims 1to 6 for the prevention and treatment of adenocarcinoma of the duodenum, ileum, colon, and rectum.

**9.** A pharmaceutical composition comprising a 9α-estra-1,3,5(10)-triene derivatives of formula (I) according to any one of the claims 1to 6 for the prevention and treatment of duodenal or colorectal adenoma.
